Europäisches Patentamt

European Patent Office

Office européen des brevets

(19)

(11) Numéro de publication: **0 223 628**
A1

(12) **DEMANDE DE BREVET EUROPEEN**

(21) Numéro de dépôt: 86402020.1

(22) Date de dépôt: 16.09.86

(51) Int. Cl.⁴: **C 12 P 7/40, C 12 P 7/52**

(30) Priorité: 18.09.85 FR 8513817

(43) Date de publication de la demande: 27.05.87
Bulletin 87/22

(84) Etats contractants désignés: AT BE CH DE FR GB IT LI LU NL SE

(71) Demandeur: Société Chimique des Charbonnages S.A., Tour Aurore Place des Reflets, F-92080 Paris La Défense 2 Cédex 5 (FR)

(72) Inventeur: Pacaud, Stéphane, 15, rue Pech David, F-31400 Toulouse (FR)
Inventeur: Goma, Gérard, 20 Rue du Salas, F-31520 Ramonville (FR)

(74) Mandataire: Fourquet, Antoinette, SOCIETE CHIMIQUE DES CHARBONNAGES S.A. Service Propriété Industrielle B.P. No. 57, F-62670 Mazingarbe (FR)

(54) Procédé de condensation d'acides carboxyliques saturés par fermentation anaérobique du méthanol.

(57) Procédé de condensation d'acides carboxyliques saturés présentant n atomes de carbone à partir de l'acide acétique produit par voie anaérobique dans un milieu constitué de méthanol, d'un réactif capable de généner du gaz carbonique et de microorganismes capables de convertir biologiquement le méthanol (Eubacterium Limosum, Butyribacterium Melicylotrophicum). On ajoute dans le milieu des sels d'acides carboxyliques saturés présentant n atomes de carbone en quantité telle qu'ils présentent dans le milieu une molarité au moins égale à 0,4/n.

ACTORUM AG

La présente invention concerne un procédé de condensation d'acides carboxyliques saturés, elle a plus particulièrement pour objet un procédé de condensation d'acides carboxyliques saturés présentant n atomes de carbone avec l'acide acétique, mettant en oeuvre du méthanol et une souche de microorganismes.

Le méthanol est un alcool facilement disponible dans l'industrie chimique et qui a besoin d'être transformé pour conduire à des produits à plus forte valeur ajoutée. Plusieurs essais ont été réalisés afin de le valoriser et le transformer par voie biochimique à l'aide de microorganismes en produits présentant des applications plus intéressantes. C'est ainsi qu'il est connu que le méthanol peut être converti en l'absence d'oxygène et à l'aide de microorganismes particuliers, en acide acétique et en acide butyrique selon la réaction :

$$\text{Méthanol} + CO_2 \xrightarrow{\text{microorganismes}} \text{Acide acétique} + \text{Acide butyrique} + \text{microorganismes}$$

On obtient donc un mélange d'acides accompagné parallèlement d'une production accrue de microorganismes.

Les microorganismes qui ont été décrits jusqu'alors sont en particulier :

- l'EUBACTERIUM LIMOSUM (GENTHNER et col 1981 Applied and Env. Microbiology 42 : 12-19)

- le BUTYRIBACTERIUM·METHYLOTROPHICUM (ZEIKUS et al 1982 - Journal of Bactoriology 149 : 255-263)

Tous ces microorganismes sont mis en oeuvre en présence d'un réactif capable de produire du gaz carbonique tel que des carbonates : carbonate acide de potassium ($CO_3HK$), carbonate de potassium ($CO_3K_2$) ou carbonate de sodium ($CO_3Na_2$).

La mise en oeuvre de ces microorganismes permet d'obtenir en présence d'un réactif capable de générer du gaz carbonique, un mélange d'acide acétique et d'acide butyrique.

Si l'on désire obtenir des acides carboxyliques supérieurs à plus grande valeur ajoutée à l'aide de microorganismes on voit donc qu'il n'est pas possible d'y parvenir en mettant en oeuvre des conditions opératoires classiques.

2

Le besoin se fait donc sentir de mettre au point un procédé permettant d'obtenir par voie anaérobique à partir de méthanol des acides carboxyliques supérieurs.

La présente invention concerne un procédé de condensation d'acides carboxyliques présentant n + 2 atomes de carbone avec l'acide acétique produit par voie anaérobique dans un milieu constitué de méthanol, d'un réactif capable de générer du gaz carbonique et de microorganismes capables de convertir biologiquement le méthanol caractérisé par le fait que l'on ajoute dans le milieu des sels d'acides carboxyliques saturés comportant n atomes de carbone, en quantité telle que leur molarité soit au moins égale à 0,4/n.

Selon une autre caractéristique du procédé de l'invention la réaction est réalisée à pH compris entre 6,5 et 7,8 et de préférence compris entre 7,2 et 7,6.

Les sels d'acides carboxyliques saturés présentant n atomes de carbone sont choisis à titre d'exemple mais de façon non limitative parmi les sels des acides acétique, propionique, butyrique. De préférence les sels sont les sels de sodium, de potassium ou d'ammonium. Leur mise en oeuvre permet de préparer les produits suivants : à partir du sel de l'acide acétique on prépare le sel de l'acide butyrique, à partir du sel de l'acide propionique on prépare le sel de l'acide valérique, à partir du sel de l'acide butyrique on prépare le sel de l'acide caproïque. De tels résultats illustrent bien le fait que le sel de l'acide carboxylique ajouté dans le milieu se condense sur l'acide acétique formé biologiquement à partir du substrat constitué de méthanol, de microorganismes et d'un réactif capable de générer du gaz carbonique. Le procédé de la présente demande présente la possibilité non seulement de fabriquer des sels d'acides carboxyliques supérieurs mais par rapport aux techniques connues qui ne mettent pas les conditions opératoires de l'invention, il permet d'augmenter les vitesses de réaction.

La mise en oeuvre du procédé de l'invention est réalisée de façon connue en utilisant un substrat constitué de méthanol, d'un réactif capable de générer du gaz carbonique, de microorganismes capables de transformer biologiquement le méthanol et d'un milieu de culture. De façon connue les solutions de méthanol ont une concentration comprise entre 1 et 20 grammes par litre et de préférence comprise entre 3 et 8 grammes par litre. Les réactifs capables de générer du gaz carbonique sont choisis parmi le carbonate acide de potassium, le carbonate de potassium ou le carbonate de sodium : ils sont utilisés en solution en quantité comprise entre 50 et 200% en poids par rapport au méthanol. Le milieu de culture utilisé est de façon connue constitué de divers éléments classiques utilisés lorsqu'on réalise des réactions avec des microorganismes : un tel milieu est par exemple décrit dans la thèse de SAMAIN - 1983 - Thèse INRA - Villeneuve d'Ascq - Faculté de Lille.

Les microorganismes mis en oeuvre selon le procédé de la présente invention sont choisis en particulier parmi l'EUBACTERIUM LIMOSUM $B_2$.

Après la fin de la réaction le milieu est acidifié par un acide minéral de façon à libérer les acides carboxyliques formés : ces acides sont identifiés par chromatographie analytique en phase gazeuse.

Les exemples suivants illustrent la présente invention :

**EXEMPLE 1**

On utilise un milieu de culture dont la description est donnée dans la thèse de SAMAIN citée ci-dessus. On ajoute à ce milieu une solution contenant 3 grammes/litre de méthanol et 5 grammes/litre de carbonate acide de potassium présentant une concentration de 5 grammes/litre. On introduit dans ce milieu 50 millilitres d'un inoculum constitué de EUBACTERIUM LIMOSUM $B_2$ (décrit par SAMAIN : thèse INRA - Villeneuve d'Asq (1983) - Lille), à 0,3 gramme/litre de matière sèche, le pH étant ajusté à la valeur de 7,4. On ajoute différentes quantités de sel de sodium de l'acide acétique. Le tableau 1 résume les résultats obtenus.

4

Les valeurs négatives d'acide acétique signifient que l'acide acétique a été consommé pour la production d'acide butyrique.

**EXEMPLE 2**

L'exemple 1 est répété en utilisant les mêmes microorganismes mais en mettant en oeuvre une solution de méthanol présentant une concentration de 2,5 grammes/litre, et en remplaçant l'acide acétique par l'acide butyrique.

Le tableau 2 résume les résultats obtenus.

**EXEMPLE 3**

L'exemple 1 est répété mais en remplaçant l'acide butyrique par l'acide valérique.

Le tableau 3 résume les résultats obtenus.

**TABLEAU 1**

| CONDITIONS INITIALES | | CONDITIONS FINALES - PRODUCTIONS | | |
|---|---|---|---|---|
| Substrats | Acide Acétique $g.l^{-1}$ | Acide Acétique $g.l^{-1}$ | Acide Butyrique $g.l^{-1}$ | Rendement en poids par rapport au méthanol consommé |
| Méthanol | 0,30 | 1,59 | 1,59 | 53% |
| 3,0 $g.l^{-1}$ | 4,90 | 0,34 | 2,28 | 76% |
| | 8,40 | -0,10 | 2,32 | 77% |
| $KHCO_3$ | 15,1 | -0,60 | 2,65 | 88% |
| 5,0 $g.l^{-1}$ | 24,4 | -2,10 | 2,83 | 94% |

## TABLEAU 2

| CONDITIONS INITIALES | | CONDITIONS FINALES - PRODUCTIONS | | |
|---|---|---|---|---|
| Substrats | Acide Butyrique $g.l^{-1}$ | Acide Acétique $g.l^{-1}$ | Acide Butyrique $g.l^{-1}$ | Acide Caproïque $g.l^{-1}$ |
| Méthanol 2,25 $g.l^{-1}$ | 0,00 | 0,85 | 1,28 | 0,00 |
| | 5,3 | 1,65 | 0,60 | 0,00 |
| | 10,7 | 1,89 | - 0,17 | 0,18 |
| KHCO$_3$ 5,0 $g.l^{-1}$ | 15,9 | 2,14 | - 0,48 | 0,22 |
| | 21,2 | 1,80 | - 0,74 | 0,80 |

**TABLEAU 3**

| CONDITIONS INITIALES | | CONDITIONS FINALES - PRODUCTIONS | | |
|---|---|---|---|---|
| Substrats | Acide Proprionique $g.l^{-1}$ | Acide Acétique $g.l^{-1}$ | Acide Butyrique $g.l^{-1}$ | Acide Valérique $g.l^{-1}$ |
| Méthanol 3,02 $g.l^{-1}$ | 7,4 | 1,44 | 0,95 | 1,00 |
| $KHCO_3$ 5,0 $g.l^{-1}$ | 14,8 | 1,80 | 0,50 | 1,50 |
| | 21,2 | 1,56 | 0,25 | 1,84 |

7

0223628

8

## REVENDICATIONS

1. Procédé de préparation d'acides carboxyliques saturés présentant n + 2 atomes de carbone à partir de l'acide acétique produit par voie anaérobie dans un milieu contenant du méthanol, d'un réactif susceptible de générer du gaz carbonique et de microorganismes capables de convertir biologiquement le méthanol, caractérisé en ce que l'on ajoute dans le milieu des sels d'acides carboxyliques saturés présentant n atomes de carbone en quantité telle qu'il présente dans le milieu une molarité au moins égale à $\frac{0,4}{n}$.

2. Procédé selon les revendications 1 et 2, caractérisé en ce que la réaction est réalisée à pH compris entre 6,5 et 7,8.

0223628

Numéro de la demande

EP 86 40 2020

**Office européen des brevets**

## RAPPORT DE RECHERCHE EUROPEENNE

### DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| Y | US-A-4 425 432 (J. ZEIKUS) <br> * Revendications; colonnes 7-10 * | 1 | C 12 P 7/40 <br> C 12 P 7/52 |
| Y | US-A-4 377 638 (M. BRYANT) <br> * Revendications * | 1 | |
| A | BIOTECHNOLOGY AND BIOENGINEERING, vol. 25, no. 4, avril 1983, pages 991-998, John Wiley & Sons, Inc., New York, US; R. DATTA et al.: "Methanol bioconversion by Butyribacterium methylotrophicum - batch fermentation yield and kinetics" <br> * En entier * | 1 | |

**DOMAINES TECHNIQUES RECHERCHES (Int. Cl.4)**

C 12 P

Le présent rapport de recherche a été établi pour toutes les revendications

| Lieu de la recherche | Date d'achèvement de la recherche | Examinateur |
|---|---|---|
| LA HAYE | 12-12-1986 | DELANGHE L.L.M. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

OEB Form 1503 03 82